# EUROPÄISCHE PATENTANMELDUNG

(11) **EP 1 083 214 A2**
(43) Veröffentlichungstag der Anmeldung: **14.03.2001**
(21) Anmeldenummer: 00113682.9
(22) Anmeldetag: 28.06.2000
(51) Int. Cl.: C11D 1/62, C11D 1/835, A61L 9/05

(54) **Zubereitung, insbesondere für WC-Erfrischer**

(30) Priorität: 11.08.1999 DE 19937987
(71) Anmelder: drom fragrances international KG, 82065 Baierbrunn (DE)
(72) Erfinder: Meller, Gerhard, 53879 Euskirchen (DE)
(74) Vertreter: Patentanwälte Dr. Solf & Zapf

(57) **Zusammenfassung**

Die Erfindung betrifft eine Zubereitung, insbesondere für WC-Erfrischer, umfassend Hilfs- und Duftstoffe sowie Tenside und ist dadurch gekennzeichnet, daß als Tensid wenigstens ein kationisches Tensid verwendet wird, wodurch ein klares viskoses Produkt mit antibakteriellen Eigenschaften erhalten wird, das bei Anwendung nur wenig schäumt.

## Beschreibung

Die Erfindung betrifft eine Zubereitung, insbesondere für WC-Erfrischer.

Bekannte WC-Erfrischer umfassen beispielsweise WC-Steine, die in Körbchen unter den Beckenrand gehängt bzw. direkt in das Spülbecken eingebracht werden, um beim Spülvorgang die Inhaltsstoffe der WC-Erfrischer in das Becken der Toilette zu spülen. Andere bekannte WC-Erfrischer bestehen aus Gelen oder umfassen Einhängesysteme, die mit einem Nachfüllbehältnis für eine flüssige Zubereitung versehen sind.

Die bekannten Zubereitungen für WC-Erfrischer können Hilfs- und Duftstoffe ebenso wie Mittel zur Verhinderung von Kalkablagerungen enthalten.

Aus der EP 0 775 741 A1 sind flüssige Zubereitungen für WC-Erfrischer bekannt, welche anionische und/oder nichtionische Tenside enthalten.

Die Verwendung anionischer Zubereitungen ist jedoch einerseits mit dem Nachteil verbunden, daß je nach Typ der Toilette beim Spülvorgang eine hohe Schaumentwicklung entstehen kann und des weiteren dürfen solche anionische Zubereitungen keine kationischen Tenside als bakterizide und fungizide Mittel enthalten. Bei Zubereitungen, die aus nichtionischen Tensiden bzw. aus einer Mischung aus nichtionischen und anionischen Tensiden bestehen, tritt weiterhin ein Problem der Lagerbeständigkeit auf, da eine längere Lagerzeit zu einem Viskositätsanstieg führen kann, wodurch sich Dosierprobleme und auch Schaumprobleme ergeben können.

Der Erfindung liegt daher die Aufgabe zugrunde, eine klare Zubereitung, insbesondere für WC-Erfrischer zu schaffen, die nur schwach schäumt und desinfizierend wirkt.

Diese Aufgabe wird durch die im Anspruch 1 angegebene Zubereitung gelöst. Die Unteransprüche 2 bis 10 geben vorteilhafte Ausgestaltungen der Zubereitung nach Anspruch 1 an.

Die Ansprüche 12 und 13 beschreiben eine besonders wirkungsvolle und günstige Zubereitung für WC-Erfrischer.

Gegenstand der Erfindung ist daher eine Zubereitung, insbesondere für WC-Erfrischer, die neben an sich bekannten Hilfs- und Duftstoffen wenigstens ein kationisches Tensid enthält.

Besonders günstige Ergebnisse liefern kationische Tenside, die ausgewählt sind aus:
N-Aklyldimethylbenzylammoniumchlorid mit 8 bis 18 C-Atomen;
N-Dialkylmethylbenzylammoniumchlorid mit 12 bis 18 C-Atomen;
N-Tetradecyldimethylbenzylammoniumchlorid;
N-Alkyldimethylethylbenzylammoniumchlorid mit 12-14 C-Atomen;
Octyldecyldimethylammoniumchlorid;
Dioctyldimethylammoniumchlorid;
Didecyldimethylammoniumchlorid;

Zubereitungen für WC-Erfrischer, welche die obengenannten kationischen Tenside enthalten, bilden eine klare viskose Flüssigkeit mit antibakteriellen Eigenschaften, welche bei Verwendung in einem Einspülsystem in der Toilette kaum Schaum bildet.

Das kationische Tensid ist in der Zubereitung in einer Menge von 5 bis 50 Gew.-%, vorzugsweise 5 bis 30 Gew.-%, insbesondere 5 bis 20 Gew.-% enthalten.

Zur Geruchsverbesserung können die erfindungsgemäßen Zubereitungen beispielsweise Parfümöle enthalten, wobei sich als zusätzliche Emulagatoren für die lipophilen Bestandteile und zum Aufbau eines Teiles der Strukturviskosität nichtionische Tenside vorteilhaft einsetzen lassen. Durch die Verwendung nichtionischer Tenside läßt sich das Anschäumverhalten verbessern, ohne daß sich ein unerwünschter stabiler Schaum aufbaut.

Als nichtionische Tenside werden vorzugsweise Fettsäureglyceride, Sorbitanester oder auch Fettalkoholethoxylate basierend auf Fettsäuren, Fettalkoholen oder Fettsäurederivaten mit 11 bis 18 C-Atomen und einem Oxethylierungsgrad von 2-20 Mol EO, ebenso wie Fettsäurealkanolamide auf Basis von Kokosfettsäure mit Diethanolamin verwendet.

Beispiele für in der Erfindung verwendbare nichtionische weitere Tenside sind Sorbitanmonolaurat, Sorbitansesquioleat, Fettalkohol C12 bis C14 mit 2 - 9 Mol EO, Polyoxyethylen-24-glycerinmonostearat, Kokosfettsäurediethanolamid und Isotridecylalkoholpolyglykolether mit 5 bis 8 Mol EO.

Die nichtionischen Tenside sind in einer Menge von 1 bis 10 Gew.-%, vorzugsweise 3 bis 7 Gew.-% in der Zubereitung enthalten.

Überraschenderweise wurde gefunden, daß die erfindungsgemäßen Zubereitungen, insbesondere für WC-Erfrischer auch im pH-Bereich von z.B. pH 2 bis pH 9 zu klaren Lösungen führen, und beim Spülvorgang keine hohe Schaumentwicklung bzw. Bildung stabiler Schäume auftritt.

Vorzugsweise enthält die Zubereitung NaOH oder KOH.

Im folgenden wird die Erfindung anhand von Beispielen erläutert, wobei sich die angegebenen Prozentzahlen auf das Gewicht beziehen, und der Fachmann weiß, daß diese Rezeptur im Rahmen des fachmännischen Könnens modifiziert werden kann.

| Beispiel 1: | |
|---|---|
| Alkyldimethylbenzylammoniumchlorid | 1-50 Gew-%, |
| Zitronensäure | 1-10 Gew-%, |
| Polyolfettsäureester | 1-5 Gew-%, |
| Parfum | 2-15 Gew.-%, |
| q.s. Farbe | |
| Wasser auf | 100 Gew.-% |

| Beispiel 2: | |
|---|---|
| Lauryldiethylentriaminoessigsäure | 1-30 Gew.-%, |
| 2-Hydroxypropionsäure | 1-30 Gew.-%, |
| Fettaminpolyethylenglycolether | 1-5 Gew.-%, |
| Kalium Natrium Cumolsulfonat | 1-5 Gew.-%, |
| Kaliumhydroxid | 0,3-2 Gew.-%, |
| Parfum | 2-15 Gew.-%, |
| q.s. Farbe | |
| Wasser auf | 100 Gew.-% |

## Patentansprüche

1. Zubereitung, insbesondere für WC-Erfrischer, umfassend Hilfs- und Duftstoffe sowie Tenside,
**dadurch gekennzeichnet,**
daß die Zubereitung wenigstens ein kationisches Tensid enthält.

2. Zubereitung nach Anspruch 1,
**dadurch gekennzeichnet,**
daß das kationische Tensid ausgewählt ist aus:
N-Aklyldimethylbenzylammoniumchlorid mit 8 bis 18 C-Atomen;
N-Dialkylmethylbenzylammoniumchlorid mit 12 bis 18 C-Atomen;
N-Tetradecyldimethylbenzylammoniumchlorid;
N-Alkyldimethylethylbenzylammoniumchlorid mit 12-14 C-Atomen;
Octyldecyldimethylammoniumchlorid;
Dioctyldimethylammoniumchlorid;
Didecyldimethylammoniumchlorid;

3. Zubereitung nach einem der Ansprüche 1 oder 2,
**dadurch gekennzeichnet,**
daß die Zubereitung wenigstens ein nichtionisches Tensid enthält.

4. Zubereitung nach Anspruch 2,
**dadurch gekennzeichnet,** daß
das nichtionische Tensid ausgebildet ist aus Fettsäureglyceriden, Sorbitanestern und Fettalkoholethoxylaten basierend auf Fettsäuren, Fettalkoholen oder Fettsäurederivaten mit 11 bis 18 C-Atomen und einem Oxethylierungsgrad von 2-20 Mol EO sowie aus Fettsäurealkanolamiden auf Basis von Kokosfettsäure mit Diethanolamin.

5. Zubereitung nach Anspruch 4,
**dadurch gekennzeichnet,**
daß das nichtionische Tensid, Sorbitanmonolaurat, Sorbitansesquioleat, Fettalkohol C12 bis C14 mit 2 - 9 Mol EO, Polyoxyethylen-24-glycerinmonostearat, Kokosfettsäurediethanolamid und Isotridecylalkoholpolyglykolether mit 5 bis 8 Mol EO ist.

6. Zubereitung nach einem der Ansprüche 1 bis 5,
**dadurch gekennzeichnet,**
daß die Zubereitung auf einen pH-Wert zwischen 2 bis 9 eingestellt ist.

7. Zubereitung nach Anspruch 6,
**dadurch gekennzeichnet,**
daß sie auf einen pH-Wert von 3 bis 9 eingestellt ist.

8. Zubereitung nach einem der Ansprüche 1 bis 7,
**dadurch gekennzeichnet,**
daß sie Zitronensäure enthält.

9. Zubereitung nach einem der Ansprüche 1 bis 8,
dadurch gekennzeichnet, daß sie NaOH oder KOH enthält.

10. Zubereitung nach einem der Ansprüche 1 bis 9,
**dadurch gekennzeichnet,**
daß das kationische Tensid in einer Menge von 5 bis 40 Gew.-% enthalten ist.

11. Zubereitung nach einem der Ansprüche 1 bis 10,
**dadurch gekennzeichnet,**
daß sie ein Parfümöl enthält.

12. Zubereitung, insbesondere für WC-Erfrischer,
umfassend:
| | |
|---|---|
| Alkyldimethylbenzylammoniumchlorid | 1-50 Gew-%, |
| Zitronensäure | 1-10 Gew-%, |
| Polyolfettsäureester | 1-5 Gew-%, |
| Parfum | 2-15 Gew.-%, |
| q.s. Farbe | |
| Wasser auf | 100 Gew.-% |

13. Zubereitung, insbesondere für WC-Erfrischer,
umfassend:
| | |
|---|---|
| Lauryldiethylentriaminoessigsäure | 1-30 Gew.-%, |
| 2-Hydroxypropionsäure | 1-30 Gew.-%, |
| Fettaminpolyethylenglycolether | 1-5 Gew.-%, |
| Kalium Natrium Cumolsulfonat | 1-5 Gew.-%, |
| Kaliumhydroxid | 0,3-2 Gew.-%, |
| Parfüm | 2-15 Gew.-%, |
| q.s. Farbe | |
| Wasser auf | 100 Gew.-% |
